# EUROPEAN PATENT APPLICATION

(11) **EP 4 350 350 A1**
(43) Date of publication of application: **10.04.2024**
(21) Application number: 22816095.8
(22) Date of filing: 31.05.2022
(51) Int. Cl.: G01N 33/49, G01N 33/68

(54) **METHOD FOR ASSESSING RISK OF HEMORRHAGIC STROKE USING SOLUBLE CLEC2**

(30) Priority: 31.05.2021 JP 2021091607
(71) Applicant: PHC Corporation, Toon-shi Ehime 791-0395 (JP)
(72) Inventor: WADA, Hideo, Yokkaichi-shi, Mie 510-8561 (JP); KAWAMURA, Masahide, Toon-shi, Ehime 791-0395 (JP)
(74) Representative: Gille Hrabal Partnerschaftsgesellschaft mbB Patentanwälte
(86) International application number: PCT/JP2022/022077
(87) International publication number: WO 2022/255349

(57) **Abstract**

In the treatment of hemorrhagic stroke, provided is a means for conveniently complementing the diagnosis by blood tests. The method comprises a step of measuring (or determining) the concentration of soluble CLEC2 (or a value obtained by dividing the soluble CLEC2 concentration by platelet counts) present in blood collected from a patient suspected of having hemorrhagic stroke or a patient diagnosed with hemorrhagic stroke.

## Description

### TECHNICAL FIELD

The present invention relates to a diagnostic method and a monitoring method for hemorrhagic stroke using soluble CLEC2.

### BACKGROUND ART

Stroke is a group of diseases that includes ischemic stroke which is caused by a clogged blood vessel in the brain, i.e., cerebral infarction, hemorrhagic stroke which is caused by a rupture of blood vessel in the brain, i.e., cerebral hemorrhage, and subarachnoid hemorrhage, and is the second leading cause of death worldwide, after ischemic heart disease. Stroke is not only a major cause of death, but even after patients survive, they are often left with severe aftereffects and require nursing care, which places a heavy burden on patients and society. Therefore, a system that enables prevention, early diagnosis, prompt response after diagnosis, and care and risk management by a wide range of medical personnel is needed.

Hemorrhagic strokes include subarachnoid hemorrhage caused by a ruptured aneurysm in an artery on the surface of the brain, and cerebral hemorrhage, which occurs when a small artery in the brain breaks.

Subarachnoid hemorrhage is a hemorrhage of blood vessels in the space between the brain and the membrane on the surface of the brain called the arachnoid membrane. The symptoms of subarachnoid hemorrhage are almost always severe headaches, described as "headache like being hit by a baseball bat" or "headache that I have never experienced before". The mortality rate of subarachnoid hemorrhage is as high as 30%, and it is a very serious acute disease that must be treated immediately and diagnosed as soon as possible.

The diagnosis of subarachnoid hemorrhage can be confirmed relatively easily by CT scan (hereinafter abbreviated as CT) imaging as soon as the patient presents with typical clinical symptoms. The sensitivity of CT imaging (positive rate in patients) is said to be 98% to 100% in the first 12 hours after the onset of the disease, and the diagnostic ability is very good, allowing the diagnosis on this test alone. However, if there is no CT scanner, the patient must be transported to a facility with a CT scanner, and in some areas, there are only a few hospitals with a CT scanner or a CT scanner with poor sensitivity. In addition, 10% to 15% of subarachnoid hemorrhage cases are mild cases who come to the hospital by foot, and in these cases, the hemorrhage may not be confirmed by CT images because of the long time that has passed since the onset or because the bleeding is mild. In such cases, a needle is inserted into the lumbar spine to collect cerebrospinal fluid, and the presence or absence of blood in the fluid is checked. The sensitivity of this test (the positive rate in patients) is almost 100%, which is very good to complement negative CT images, but it is an invasive and burdensome test for patients because of the needle puncture in the lumbar spine.

In the past, D-dimer measurement and other tests were proposed to diagnose subarachnoid hemorrhage by blood tests. However, in order to complement the cerebrospinal fluid measurement, the sensitivity (positive rate in patients) must be almost 100%, but no such sensitivity has been achieved not only in the cerebrospinal fluid measurement but also in conventional blood test items, and none are actually in use at present.

Even if subarachnoid hemorrhage can be saved in the early stage after the onset, complications such as subsequent cerebral vasospasm and the like are also a major therapeutic problem. Since cerebral vasospasm often occurs from about 4 days to 2-3 weeks after the onset, it is very important to monitor the patient's progress. In the treatment of subarachnoid hemorrhage, it is important not only to diagnose whether or not the patient has a subarachnoid hemorrhage, but also to predict whether or not the patient will have serious complications such as cerebral vasospasm and the like after the onset.

A number of biomarkers are known to predict complications of subarachnoid hemorrhage using cerebrospinal fluid samples. Many biomarkers in cerebrospinal fluid, such as endothelin, TNF-α, IL-8, IL-6, thrombin-antithrombin complex, HMGB1, and the like, have been reported to be associated with complications in patients with subarachnoid hemorrhage, but these biomarkers have not been put to practical use because they require cerebrospinal fluid samples from the lumbar spine. Even if it were done, it is not realistic to use it to observe changes over time, since it is invasive and burdensome to the patient, and the sample is collected only once.

On the other hand, cerebral hemorrhage is an acute disease in which a blood vessel in the brain breaks for some reason, causing bleeding in the brain. Blood clots from the hemorrhage press on the brain or cause swelling of the brain, resulting in brain dysfunction. Symptoms vary depending on the location and amount of hemorrhage. Symptoms may be sensory symptoms such as headache, nausea, and vomiting, or motor symptoms such as paralysis of the hands and feet, numbness, and gait disturbance, and are difficult to distinguish from cerebral infarction because some symptoms are similar to those of cerebral infarction. In addition, the amount of blood loss may be life-threatening. As with subarachnoid hemorrhage, the diagnosis is often made by CT scan.

Cerebral hemorrhage can be treated by lowering blood pressure or the like after diagnosis if the bleeding stops, but if it does not, a hematoma in the brain will increase and the symptoms will worsen. Although the growth of the hematoma can be confirmed by repeated CT imaging, as mentioned above, it is often difficult to perform frequent CT imaging examinations using a CT scanner with a certain level of accuracy.

In addition, although S100β, GFAP, and the like are known as biomarkers for monitoring the symptoms of cerebral hemorrhage at the research level, their clinical usefulness is low and none are used in actual clinical practice, and there is no test that can be conveniently monitored by blood tests or other means.

C-type lectin-like receptor 2 (CLEC2) was identified on platelets as a receptor for the platelet-activating snake venom rhodocytin. CLEC2 is expressed almost exclusively on platelets and megakaryocytes in humans, and is therefore a platelet-specific molecule. It was reported that CLEC2 is released into the blood as soluble CLEC2 (soluble CLEC-2, hereafter abbreviated as sCLEC2) when platelets are activated (Patent literature 1, Non-patent literature 1).

### CITATION LIST

### PATENT LITERATURE

[Patent literature 1] JP 6078845 B
[Patent literature 2] Japanese Patent Application No. 2020-032797
[Patent literature 3] Japanese Patent Application No. 2021-003671

### NON-PATENT LITERATURE

[Non-patent literature 1] F. Kazama et al., Platelets 2015; 26(8): 711-719
[Non-patent literature 2] Y. Yamashita et al., Thrombosis Research 178 (2019) 54-58

### SUMMARY OF INVENTION

### TECHNICAL PROBLEM

Hemorrhagic stroke has a high mortality rate, and even if death is avoided, it is a serious acute-stage disease that may leave behind sequelae, so it is extremely important to diagnose and begin treatment as soon as possible. If a patient presents with typical symptoms, visits a medical institution within a short time after the onset, and undergoes imaging tests such as CT, MRI, or the like to confirm hemorrhage in the brain, the diagnosis can be made on that basis alone. However, in some cases, such as mild cases with a small amount of bleeding, cases with a mild disease that reach a specialized medical institution after a long time, and the like, hemorrhage may not be confirmed by CT images or the like. In addition, medical institutions with highly sensitive CT and MRI may be limited depending on national and regional conditions. In such cases, patients with subarachnoid hemorrhage are subjected to the highly invasive test of cerebrospinal fluid examination by lumbar puncture. Lumbar puncture is not performed in cerebral hemorrhage, but if it cannot be confirmed by CT images or the like, it is difficult to make a precise diagnosis.

Even if death is avoided by initial treatment, subarachnoid hemorrhage has a very dangerous complication called cerebral vasospasm, and patients need to be carefully monitored by a physician for a while after the initial treatment. Although there are some biomarkers that can predict cerebral vasospasm using cerebrospinal fluid in literatures, there is no biomarker that can conveniently monitor the symptoms by blood tests that is used in actual clinical practice.

The initial treatment of cerebral hemorrhage is diagnosed by CT, MRI, and the like, followed by treatment such as antihypertensive therapy or the like, but there is no biomarker that can monitor whether the treatment is successful and hemostasis is achieved.

Therefore, a problem of the present invention is, in the treatment of hemorrhagic stroke, to provide a means to conveniently complement the diagnosis of hemorrhagic stroke by blood tests. In addition, another problem of the present invention is to provide a method to conveniently monitor the patient's conditions during the treatment for subarachnoid hemorrhage by blood tests. Still another problem of the present invention is, for cerebral hemorrhage, to provide a means to conveniently judge the effect of treatment and to monitor the patient's symptoms by blood tests.

### SOLUTION TO PROBLEM

The inventors have conducted intensive studies to solve the above-mentioned problems. As a result, we found that the blood sCLEC2 concentration in hemorrhagic stroke patients was increased in almost all patients compared to that in normal subjects, leading to the completion of the present invention. In other words, we found a method to diagnose subarachnoid hemorrhage and cerebral hemorrhage by conveniently detecting hemorrhagic stroke patients with a very high sensitivity as the positive rate of patients by blood tests.

Furthermore, we found that it is possible to monitor the condition of patients with subarachnoid hemorrhage and cerebral hemorrhage by measuring the sCLEC2 concentration over time and observing its fluctuation. This may be used to predict serious complications such as cerebral vasospasm and the like in patients with subarachnoid hemorrhage.

In order to determine hemorrhagic stroke patients from non-hemorrhagic stroke patients using sCLEC2, an appropriate threshold is required. In the present invention, we found that the hemorrhagic stroke threshold for the blood sCLEC2 concentration is preferably 137 pg/mL or less.

Based on the above, we have completed the present invention as a method for evaluating the risk of hemorrhagic stroke using sCLEC2.

The present invention provides the following:
[1] A method for evaluating a risk of hemorrhagic stroke, said method comprising: measuring a concentration of soluble CLEC2 in blood collected from a patient suspected of having hemorrhagic stroke or a patient diagnosed with hemorrhagic stroke.
[2] The method of [1] for evaluating the risk of hemorrhagic stroke in a patient suspected of having hemorrhagic stroke or a patient diagnosed with hemorrhagic stroke, said method comprising:
   (1) providing a blood sample derived from the patient;
   (2) determining the concentration of soluble CLEC2 in the sample; and
   (3) correlating the soluble CLEC2 concentration with the presence or absence of hemorrhagic stroke in the patient, or likelihood of outcome.
[3] The method of [2] for evaluating the risk of hemorrhagic stroke in a patient suspected of having hemorrhagic stroke or a patient diagnosed with hemorrhagic stroke, said step of correlating the soluble CLEC2 concentration with a presence or absence of hemorrhagic stroke in the patient, or likelihood of outcome comprising:
   evaluating whether the patient is at risk based on a change in the soluble CLEC2 concentration.
[4] The method of [2] or [3] for evaluating the risk of hemorrhagic stroke in a patient suspected of having hemorrhagic stroke or a patient diagnosed with hemorrhagic stroke,
   wherein, in the step of correlating the soluble CLEC2 concentration with hemorrhagic stroke, its threshold is 137 pg/mL.
[5] The method of [1] for evaluating the risk of hemorrhagic stroke in a patient suspected of having hemorrhagic stroke or a patient diagnosed with hemorrhagic stroke, said method comprising:
   (1) providing a blood sample derived from the patient;
   (2) determining the concentration of soluble CLEC2 in the sample;
   (3) determining platelet counts in the sample;
   (4) dividing the soluble CLEC2 concentration by the platelet counts; and
   (5) correlating the value obtained by dividing the soluble CLEC2 concentration by the platelet counts, with a presence or absence of hemorrhagic stroke in the patient.
[6] The method of [5] for evaluating the risk of hemorrhagic stroke in a patient suspected of having hemorrhagic stroke or a patient diagnosed with hemorrhagic stroke, said method comprising:
   wherein, in the step of correlating the value obtained by dividing the soluble CLEC2 concentration by the platelet counts with hemorrhagic stroke, a threshold of the value obtained by dividing the soluble CLEC2 concentration by the platelet counts is 0.7 to 1.0.
[7] The method of any one of [1] to [6], wherein the hemorrhagic stroke is either subarachnoid hemorrhage or cerebral hemorrhage.
[8] The method of any one of [1] to [7], wherein the step of determining the concentration of the soluble CLEC2 is performed by highly sensitive immunoassay, such as chemiluminescence immunoassay, electrochemiluminescence immunoassay, or fluorescence immunoassay.

Furthermore, the present invention includes:
- a method for evaluating a risk of hemorrhagic stroke, wherein a concentration of soluble CLEC2 (or a value obtained by dividing the soluble CLEC2 concentration by platelet counts) in a sample is measured (or determined);
- a method for assisting in a risk evaluation of hemorrhagic stroke, wherein a concentration of soluble CLEC2 (or a value obtained by dividing the soluble CLEC2 concentration by platelet counts) in a sample is measured (or determined);
- a method for measuring (or determining) a concentration of soluble CLEC2 (or a value obtained by dividing the soluble CLEC2 concentration by platelet counts) in a sample for a risk evaluation of hemorrhagic stroke;
- an in vitro method for evaluating a risk of hemorrhagic stroke, characterized by measuring (or determining) a concentration of soluble CLEC2 (or a value obtained by dividing the soluble CLEC2 concentration by platelet counts) in a sample;
- use of an antibody capable of detecting a concentration of soluble CLEC2 in the manufacture of a kit for a risk evaluation of hemorrhagic stroke; and
- a method for measuring (or determining) a concentration of soluble CLEC2 (or a value obtained by dividing the soluble CLEC2 concentration by platelet counts) in a sample to provide information necessary for a risk evaluation of hemorrhagic stroke.

### ADVANTAGEOUS EFFECTS OF INVENTION

The method of the present invention, measuring the concentration of sCLEC2 present in the blood of a patient suspected of having hemorrhagic stroke enables a rapid and convenient diagnosis of hemorrhagic stroke. Furthermore, in monitoring the course of treatment after the diagnosis of subarachnoid hemorrhage, it is also expected to improve the accuracy of judging the effectiveness of treatment and predicting complications. For patients with cerebral hemorrhage, it is possible to conveniently judge the effectiveness of treatment by measuring the concentration of sCLEC2 present in the blood of the patients.

### BRIEF DESCRIPTION OF THE DRAWINGS

[Fig. 1] Figure 1 is a standard curve prepared using a human sCLEC2 protein as a standard.
[Fig. 2] Figure 2 is a graph in which the concentrations of sCLEC2 in plasma were compared between subarachnoid hemorrhage patients and normal subjects.
[Fig. 3] Figure 3 is a graph in which the concentrations of sCLEC2 in plasma were compared between cerebral hemorrhage patients and normal subjects.
[Fig. 4] Figure 4 is a graph of an example of temporal monitoring in a subarachnoid hemorrhage case.
[Fig. 5] Figure 5 is a graph of an example of temporal monitoring in a cerebral hemorrhage case.

### DESCRIPTION OF EMBODIMENTS

Hereinafter, the embodiments of the present invention will be explained in detail, but the embodiments of the use are not limited thereto.

The term "CLEC2" as used herein is a platelet-activating receptor belonging to a C-type lectin family, which is usually present on the membrane of platelets and is released into the blood upon platelet activation. The term "soluble CLEC2 (sCLEC2)" as used herein means CLEC2 or CLEC2-derived molecules that are released from such platelets and detected in blood.

sCLEC2 includes proteins with molecular weights of approximately 40 kDa, approximately 32 kDa, approximately 25 kDa, and the like in SDS-polyacrylamide gel electrophoresis (SDS-PAGE) under reducing conditions (Non-patent literature 1). The proteins with molecular weights of approximately 40 kDa and approximately 32 kDa are present on the membrane surface of platelets, and it is presumed to be released in the form of being included in microparticles produced upon platelet activation. These are thought to have sugar chains attached to them. On the other hand, the protein with a molecular weight of approximately 25 kDa are thought to be cleaved by proteases and released from platelets upon platelet activation. In the present invention, the amount of sCLEC2 as described above is measured. sCLEC2 can be detected together as proteins with molecular weights of approximately 40 kDa, approximately 32 kDa, and approximately 25 kDa, or it can be detected only as a protein with a molecular weight of approximately 25 kDa.

As the concentration of sCLEC2 used in the present invention, the concentration of sCLEC2 may be used alone, or preferably, the value obtained by dividing the sCLEC2 concentration by platelet counts (hereinafter sometimes referred to as C2PAC index) may be used. In the present specification, unless otherwise specified, the concentration of sCLEC2 is interpreted to include both the case of using the sCLEC2 concentration and the case of dividing the sCLEC2 concentration by platelet counts.

When the sCLEC2 concentration is divided by platelet counts for the diagnosis of hemorrhagic stroke, the platelet counts are usually measured using an automated hemocytometer (blood count meter), but can also be counted using a hemocytometer plate and a microscope.

The sCLEC2 concentration in plasma is expressed in, for example, pg/mL, and the platelet counts in blood are expressed in, for example, 1,000 platelets/mm³, and it is preferable to use the C2PAC index calculated using these values. The concentration of sCLEC2 used herein can be expressed in ng/mL, ng/L, or any other arbitrary unit, and the platelet counts can be expressed in 10,000 platelets/mm³ or any other arbitrary unit, but uniform units should be used for comparison. By using various units, sCLEC2 concentration/platelet counts can take various values, but they are essentially the same concept.

It can be expected that the calculation of the ratio is often performed using measured values from clinical laboratory equipment that measures the sCLEC2 concentration and measured values from a blood count meter that measures platelet counts. Although it is preferable in daily practice to perform this calculation automatically on a hospital laboratory system, a hospital system, an electronic medical record system, or the like that is connected to both analyzers, it is also possible to construct a system that connects the data of the two analyzers, or to construct a machine that can simultaneously measure the sCLEC2 concentration and platelet counts. Furthermore, the ratio may also be computed manually using both data.

Furthermore, the correlation between the sCLEC2 concentration in plasma or sCLEC2 concentration/platelet counts and the degree of platelet activation or various diseases may be used, for example, as a threshold for judgment, or as original data or statistical processing data to calculate the threshold for judgment.

The diagnosis of hemorrhagic stroke by dividing the sCLEC2 concentration in plasma by platelet counts in blood and calculating the amount of sCLEC2 released per platelet as an index is preferable because it is possible to evaluate the degree of platelet activation without depending on the number of platelets in blood. Specifically, for example, the sCLEC2 concentration in plasma is expressed in pg/mL (A), platelet counts in blood is expressed in 1,000 platelets/mm³ (B), and the number obtained by dividing A by B can be used as an index of platelet activation.

Samples for the measurement are preferably derived from humans, but samples derived from non-human animals may be used for the purpose of understanding the clinical conditions or the like of experimental animals. Such experimental animals are not limited, but include, for example, a guinea pig, a rat, a mouse, a dog, and the like.

A method for detecting the presence of sCLEC2 is not limited, but immunological methods using an antibody that recognizes sCLEC2 (hereinafter sometimes referred to as "anti-sCLEC2 antibody") are preferred. As the methods in which the protein is immunologically detected, any of the following methods which are known and commonly used can be used: for example, immunoassays using labeled antibodies, such as enzyme-linked immunoassay (ELISA), chemiluminescence immunoassay, electrochemiluminescence immunoassay, fluorescent immunoassay, radioimmunoassay, immunochromatography, and the like; or Western blotting, latex agglutination, immunoturbidimetric method, and the like. Among these methods, immunoassay using labeled antibodies is preferred from the viewpoints of simplicity of operation and accuracy of measurement. Since rapid results are desired for diagnosis in the emergency field, chemiluminescence immunoassay, immunochromatography, and the like are most preferred.

A sample is collected from a subject (in particular, a patient), for example, with a blood collection tube for plasma collection. Citrate blood collection tubes with low residual platelets are usually suitable, but heparin blood collection tubes or EDTA blood collection tubes can also be used. Although EDTA blood collection tubes are used for measuring platelet counts, separate blood collection tubes may be used for simultaneous blood collection. The sCLEC2 concentration in plasma is measured by using centrifuged plasma, for example, at 2000g for approximately 20 minutes, but conditions for centrifugation are not limited to this, and whole blood may also be used. The following explanation is based on, but not limited to, the measurement of the sCLEC2 concentration in plasma.

Hemorrhagic stroke for the purpose of risk evaluation in the present invention means cerebral hemorrhage that occurs inside the brain (sometimes referred to as intracerebral hemorrhage) and subarachnoid hemorrhage that occurs between the soft membrane, the inner layer of tissue covering the brain, and the arachnoid membrane, the outer layer, and does not include cerebral hemorrhage caused by head injury. Cerebral hemorrhage is known to be caused by hypertension, rupture of cerebral aneurysm, rupture of cerebral arteriovenous malformation, venous sinus occlusion, dural arteriovenous fistula, amyloid deposition, moyamoya disease, or the like, but the subject of this invention is not limited to cerebral hemorrhage caused by specific causes.

As a method of conducting risk evaluation, for example, when the sCLEC2 concentration in blood of a patient with suspected hemorrhagic stroke (subarachnoid hemorrhage or cerebral hemorrhage) is higher than that of normal subjects or a neurological disease group without hemorrhage or infarction in the brain, it can be judged that the patient is likely to have hemorrhagic stroke. Based on such a comparison, the sCLEC2 concentration can be used to diagnose hemorrhagic stroke (subarachnoid hemorrhage or cerebral hemorrhage).

For the correlation between the measured sCLEC2 concentration in a patient-derived sample and the possibility of hemorrhagic stroke, a threshold may be appropriately set and used based on the comparison of sCLEC2 concentrations in patient-derived samples and those in non-hemorrhagic stroke patient-derived samples, or the evaluation may be performed when a significant change in sCLEC2 concentration is detected from the temporal record of sCLEC2 concentrations measured before the onset in the same patient.

For the correlation between the measured sCLEC2 concentration in a patient-derived sample and the possibility of hemorrhagic stroke, a threshold may be appropriately set and used based on the comparison of sCLEC2 concentrations in patient-derived samples and those in normal subject-derived samples. For example, as a method of calculating the threshold, the analysis to create an ROC curve (Receiver Operating Characteristic Curve) from the measured sCLEC2 values in plasma can be performed, and the concentration that shows 80% or more in both sensitivity and specificity for diagnosis can be used as the threshold.

In the method of the present invention, the threshold of hemorrhagic stroke by sCLEC2 is preferable for any value of 137 pg/mL or less. As a more preferable embodiment, the same analysis can be performed when the C2PAC index, which is a value obtained by dividing the sCLEC2 concentration by the platelet counts, is used to calculate the threshold. The threshold can be set between 0.7 to 1.0 when the C2PAC index is used.

When performing risk evaluation by setting such a threshold, for example, it is possible to deny hemorrhagic stroke in a patient with headache who is suspected to have a hemorrhagic stroke, if the sCLEC2 concentration in blood is a certain threshold or less.

Since the sCLEC2 concentration in blood increases with platelet activation, it is suggested that the sCLEC2 concentration in blood is related to thrombus formation and it has been reported that the sCLEC2 concentration in blood can be used for diagnosis of cerebral infarction and myocardial infarction. However, the relationship with hemorrhagic stroke is not known, and it is surprising that the measurement of sCLEC2 concentration is useful for risk evaluation.

Conventionally, when subarachnoid hemorrhage is suspected and hemorrhagic stroke is not confirmed by imaging diagnosis, lumbar puncture/cerebrospinal fluid test is performed by inserting a needle into the lumbar spine to collect cerebrospinal fluid. However, it is a highly invasive test and cannot be easily performed by any health care worker anywhere, so it would be very convenient and useful if a blood test could replace it. If a patient is diagnosed with subarachnoid hemorrhage, it is desirable to keep the patient at rest immediately after the onset of the hemorrhage to prevent rebleeding, and to avoid invasive examinations and procedures, so it is desirable that the implementation of the present invention can be an alternative to highly invasive tests.

The main treatments for patients diagnosed with subarachnoid hemorrhage are as follows. As treatments to prevent rebleeding immediately after the onset include, in the initial stage, use of painkillers, anti-nausea drugs, and analgesics in order to rest (sedation) or control pain as much as possible (analgesia); administration of antihypertensive drugs in order to lower blood pressure (hypotension) to reduce the risk of rebleeding; and use of diuretics such as mannitol and glyceol to reduce cerebrospinal fluid to prevent damage to the brain caused by increased intracranial pressure. On the other hand, in severe cases, careful judgment is often required, such as considering the possibility of increased cerebral ischemia due to the decrease in cerebral perfusion pressure caused by hypotension.

The recommended treatment for patients diagnosed with cerebral hemorrhage is to lower systolic blood pressure in the acute phase as early as possible and maintain it for 7 days, and calcium channel antagonists or nitrates are the antihypertensive agents of choice in the acute phase of cerebral hemorrhage. If possible, the patient may be switched to oral therapy with calcium channel antagonists, angiotensin-converting enzyme (ACE) inhibitors, angiotensin II receptor blockers (ARBs), or diuretics at an early stage, depending on the patient's condition and other factors.

In both cases of subarachnoid hemorrhage and cerebral hemorrhage, the treatment requires a high level of experience and knowledge, and it is expected that the present invention will serve as an indicator to determine the treatment plan.

As another embodiment, it is also possible to monitor the patient's condition by collecting blood samples over time from a patient diagnosed with subarachnoid hemorrhage and measuring sCLEC2. Since sCLEC2 is considered to indicate the activation status of platelets, if sCLEC2 remains high, we can expect continued bleeding and hemostatic thrombus formation, and if sCLEC2 decreases, we can expect decreased hemostatic thrombus formation. Since cerebral vasospasm, a serious complication, is said to be induced by blood leakage from blood vessels, it is important and useful to monitor hemorrhage status by sCLEC2 in blood.

Another use of risk evaluation by such monitoring is to compare sCLEC2 concentrations before and after treatment of hemorrhagic stroke patients as a risk predictor. For example, if the sCLEC2 concentration decreases with treatment, platelet activation is suppressed, but if the sCLEC2 concentration remains high or increases, bleeding may continue, providing material for considering treatment options such as further antihypertensive treatment if antihypertensive treatment is not successful. In the process of monitoring, since fluctuations in blood platelet counts may be observed, monitoring with the C2PAC index is even more suitable for observing the patient's condition.

As another embodiment, it is also possible to monitor the patient's condition by collecting blood samples over time from a patient diagnosed with cerebral hemorrhage and measuring sCLEC2. Since sCLEC2 is considered to indicate the activation status of platelets, if sCLEC2 remains high, we can expect continued bleeding and hemostatic thrombus formation, and if sCLEC2 decreases, we can expect decreased hemostatic thrombus formation. In the case of continuous hemorrhage, the hematoma in the brain increases and compresses the brain, which worsens the symptoms, so it is important and useful to monitor the bleeding status by sCLEC2 in blood.

Another use of risk evaluation by such monitoring is to compare sCLEC2 concentrations before and after treatment of hemorrhagic stroke patients as a risk predictor. For example, if the sCLEC2 concentration decreases with treatment, platelet activation is suppressed, but if the sCLEC2 concentration remains high or increases, revision of antihypertensive therapy may also be considered.

In the process of monitoring, since fluctuations in blood platelet counts may be observed, monitoring with the C2PAC index is even more suitable for observing the patient's condition.

Even in the case of risk evaluation by monitoring, if the blood sCLEC2 concentration is measured in a patient who describes symptoms that may lead to hemorrhagic stroke (subarachnoid hemorrhage or cerebral hemorrhage), and the value is higher than that in normal subjects or the like, the possibility of hemorrhagic stroke can be judged to be high. In addition, by collecting blood samples from a patient diagnosed with hemorrhagic stroke over time and measuring and comparing the sCLEC2 concentrations over time, the patient's conditions can be monitored.

Even in the case of risk evaluation by monitoring, for the correlation between the measured sCLEC2 concentration in patient-derived samples and the likelihood of hemorrhagic stroke, the threshold may be appropriately set based on a comparison of the sCLEC2 concentration in patient-derived samples and that in non-hemorrhagic stroke-derived samples, or evaluation may be performed when a significant change in sCLEC2 concentration is detected based on the temporal recording of sCLEC2 concentration before the onset in the same patient.

sCLEC2 is released into the blood upon platelet activation. Conventional platelet activation markers, such as platelet factor 4 (PF4) and β-thromboglobulin (β-TG), have problems, because the physical pressure by blood collection stimulates platelets and causes nonspecific release against diseases. However, sCLEC2 has a signal transduction-dependent release mechanism that triggers platelet activation, and may be a more accurate marker for platelet activation in vivo. In addition, CLEC2 may be a platelet-specific marker with few false positives because its expression is almost limited to the platelet and megakaryocyte system in humans. Therefore, measurement of sCLEC2 enables early estimation of platelet activation status, and can be used for the diagnosis of hemorrhagic stroke and monitoring of the disease state.

### EXAMPLES

The present invention will now be further illustrated by, but is by no means limited to, the following Examples.

### <<Example 1: Measurement of sCLEC2 in human plasma>>

Plasma sCLEC2 concentrations were measured as follows.

### (Preparation of reagent for measurement and preparation of test samples)

· Sample diluent liquid: 0.1 mol/L HEPES buffer (pH 7.5) containing a preservative was used with a combination of 2% sodium octanoate and 0.5% n-octyl-β-D-glucoside (OG) to prepare the sample diluent liquid.
   The antibodies in the reagent were prepared as follows, using antibodies described in Examples of Japanese Patent No. 6078845.
· First antibody solution: A mouse monoclonal antibody (11D5) recognizing sCLEC2 was carried on magnetic latex particles (JSR Corporation) and dispersed in 0.01 mol/L MES buffer (pH 6.0) containing a preservative.
· Second antibody solution: Another mouse monoclonal antibody (11E6) recognizing sCLEC2 was labeled with alkaline phosphatase (ALP) by a maleimide method and dispersed in 0.01 mol/L MES buffer (pH 6.5) containing a preservative.
· Luminescent substrate solution: Disodium 2-chloro-5-(4-methoxyspiro{1,2-dioxetane-3,2'-(5'-chloro)-tricyclo[3.3.1.13,7]decane}-4-yl)-1-phenyl phosphate (CDP-Star (registered trademark): Applied Biosystems) was used.
· B/F washing buffer: A buffer solution containing 0.1 mol/L citric acid (pH 6.5), 0.15 mol/L NaCl, and 0.1% TritonX-100 was used.
· Test samples: A recombinant human soluble CLEC2 (hsCLEC2) protein diluted with a buffer solution (0.025 mol/L HEPES, 0.14 mol/L NaCl, 0.1% sodium octanoate, and 0.3% BSA) was used as test sample 1, and the same diluted with citrate plasma was used as test sample 2.

### (Measurement by measurement reagent)

An automated clinical testing system STACIA (registered trademark, LSI Medience Corporation) was used for the measurement.

STACIA-dedicated bottles were filled with the prepared sample diluent liquid, the first antibody solution (magnetic latex reagent), and the second antibody solution (enzyme-labeled antibody reagent), respectively, and placed in the apparatus. The measurement was performed according to the operation method of the apparatus.

Specifically, 40 µL of the sample diluent liquid was added to 10 µL of the sample and warmed at 37°C for several minutes, and then 25 µL of the first antibody solution (magnetic latex reagent) was added and warmed at 37°C for several minutes. Then, B/F separation was performed, 50 µL of the second antibody solution (enzyme-labeled antibody reagent) was added, and the mixture was warmed at 37°C for several minutes. B/F separation was performed again, 100 µL of the luminescent substrate solution was added, and the signal intensity (counts) was measured after several minutes of reaction at 37°C.

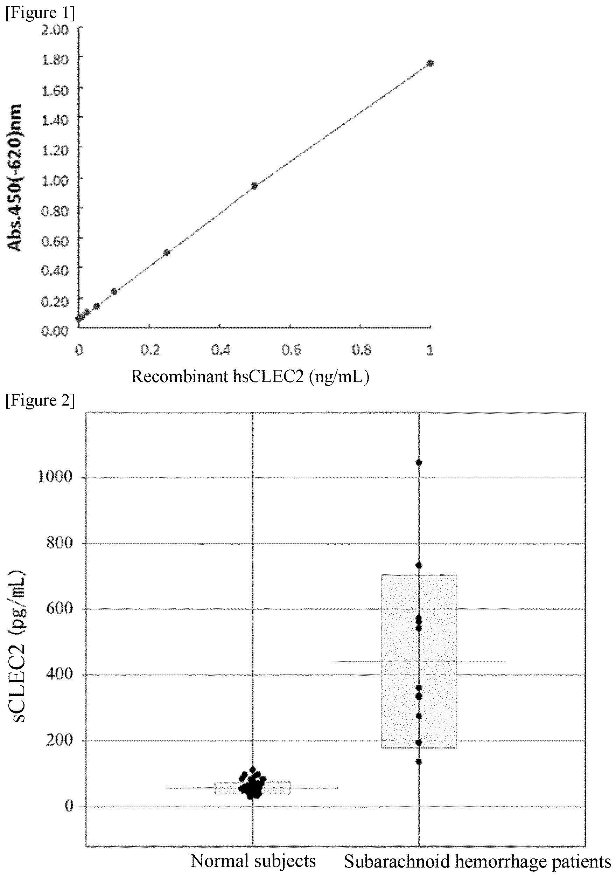

Figure 1 shows a standard curve prepared using the hsCLEC2 protein as a standard.

### <<Example 2: Measurement of sCLEC2 in plasma samples from subarachnoid hemorrhage patients, cerebral hemorrhage patients, and normal subjects>>

Plasma sCLEC2 concentrations, platelet counts, and the sCLEC2/platelet ratios (C2PAC index) were measured according to the method of Example 1, using citrate plasma obtained from patients diagnosed with subarachnoid hemorrhage or cerebral hemorrhage by outpatient consultation (Table 1 and Table 2). Additionally, sCLEC2 concentrations in normal subjects were also measured (Table 3).

**[Table 1]**

| sCLEC2 measured values and C2PAC index in patients with subarachnoid hemorrhage | | | |
|---|---|---|---|
| subarachnoid hemorrhage case | sCLEC2 (pg/mL) | Platelet counts (x1000/µL) | C2PAC index |
| 1 | 573.0 | 229 | 2.50 |
| 2 | 195.8 | 407 | 0.481 |
| 3 | 195.0 | 200 | 0.975 |
| 4 | 275.6 | 386 | 0.714 |
| 5 | 561.7 | 177 | 3.17 |
| 6 | 360.4 | 282 | 1.28 |
| 7 | 332.3 | 268 | 1.24 |
| 8 | 734.0 | 239 | 3.07 |
| 9 | 1045.8 | 360 | 2.91 |
| 10 | 138.3 | 317 | 0.436 |

**[Table 2]**

| sCLEC2 measured values and C2PAC index in patients with cerebral hemorrhage | | | |
|---|---|---|---|
| cerebral hemorrhage case | sCLEC2 (pg/mL) | Platelet counts (x1000/µL) | C2PAC index |
| 1 | 248.1 | 350 | 0.71 |
| 2 | 184.0 | 186 | 0.99 |
| 3 | 1154.0 | 497 | 2.32 |
| 4 | 696.8 | 654 | 1.07 |
| 5 | 474.2 | 223 | 2.13 |
| 6 | 263.8 | 312 | 0.85 |
| 7 | 234.8 | 284 | 0.83 |
| 8 | 703.1 | 226 | 3.11 |
| 9 | 196.4 | 222 | 0.88 |
| 10 | 168.9 | 229 | 0.74 |
| 11 | 272.2 | 191 | 1.43 |
| 12 | 147.2 | 128 | 1.15 |
| 13 | 258.5 | 267 | 0.97 |
| 14 | 408.5 | 209 | 1.95 |
| 15 | 245.1 | 221 | 1.11 |
| 16 | 161.7 | 104 | 1.55 |
| 17 | 505.1 | 186 | 2.72 |
| 18 | 365.2 | 227 | 1.61 |
| 19 | 162.8 | 286 | 0.57 |
| 20 | 216.2 | 261 | 0.83 |
| 21 | 442.0 | 282 | 1.57 |
| 22 | 199.0 | 263 | 0.76 |
| 23 | 251.8 | 251 | 1.00 |
| 24 | 169.3 | 130 | 1.30 |

**[Table 3]**

| sCLEC2 measured values in normal subjects | | | | | |
|---|---|---|---|---|---|
| Normal subject | sCLEC2 | Normal subject | sCLEC2 | Normal subject | sCLEC2 |
| 1 | 112.7 | 25 | 82.8 | 49 | 45.8 |
| 2 | 85.7 | 26 | 70.9 | 50 | 67.0 |
| 3 | 42.7 | 27 | 53.9 | 51 | 55.6 |
| 4 | 47.5 | 28 | 54.0 | 52 | 55.5 |
| 5 | 54.5 | 29 | 43.8 | 53 | 33.0 |
| 6 | 64.4 | 30 | 73.2 | 54 | 63.8 |
| 7 | 57.8 | 31 | 63.8 | 55 | 40.1 |
| 8 | 51.7 | 32 | 84.7 | 56 | 48.9 |
| 9 | 57.2 | 33 | 44.5 | 57 | 34.1 |
| 10 | 98.8 | 34 | 58.0 | 58 | 48.4 |
| 11 | 56.6 | 35 | 77.7 | 59 | 83.5 |
| 12 | 63.6 | 36 | 97.6 | 60 | 41.7 |
| 13 | 49.4 | 37 | 47.7 | 61 | 34.1 |
| 14 | 34.0 | 38 | 57.3 | 62 | 48.5 |
| 15 | 46.9 | 39 | 61.0 | 63 | 31.5 |
| 16 | 52.0 | 40 | 65.9 | 64 | 54.0 |
| 17 | 52.2 | 41 | 52.3 | 65 | 58.8 |
| 18 | 44.7 | 42 | 70.2 | 66 | 48.9 |
| 19 | 85.8 | 43 | 43.3 | 67 | 51.3 |
| 20 | 61.8 | 44 | 63.5 | 68 | 44.7 |
| 21 | 94.7 | 45 | 47.6 | 69 | 48.2 |
| 22 | 59.0 | 46 | 55.5 | 70 | 50.7 |
| 23 | 45.0 | 47 | 77.2 | 71 | 42.4 |
| 24 | 42.5 | 48 | 49.6 | | |

**[Table 4]**

| Statistical data of sCLEC2 values in normal subjects and hemorrhagic stroke patients | | | |
|---|---|---|---|
| | Normal subjects (71 cases) | Subarachnoid hemorrhage cases (10 cases) | Cerebral hemorrhage cases (24 cases) |
| Average | 57.6 | 440.9 | 338.7 |
| Standard deviation | 16.7 | 263.2 | 235.4 |
| Median | 54.6 | 348 | 250.0 |

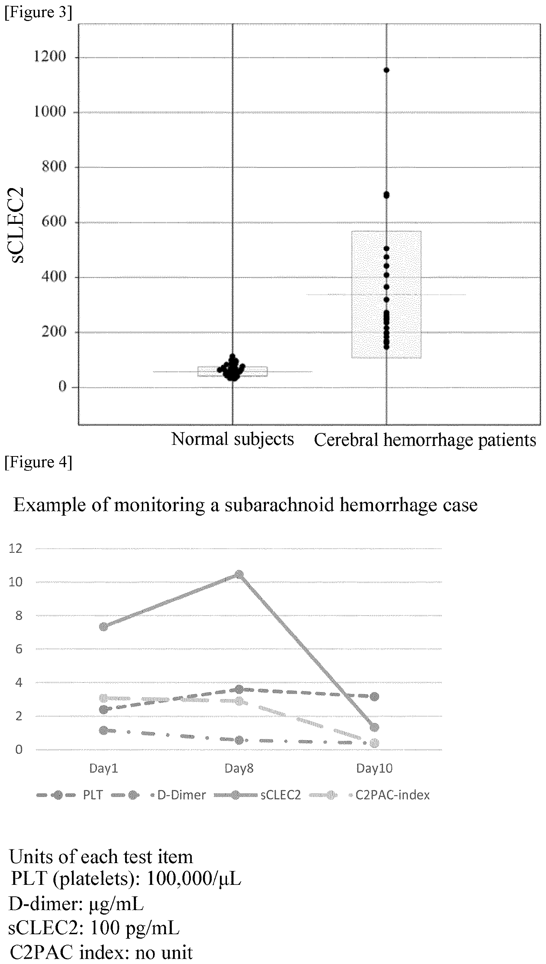

sCLEC2 measured values (pg/mL) in patients with hemorrhagic stroke were significantly higher than in normal subjects (P<0.0001) in both subarachnoid hemorrhage and cerebral hemorrhage (Table 4). Each measure value is shown in Figure 2 (subarachnoid hemorrhage) and Figure 3 (cerebral hemorrhage).

The upper limit of the C2PAC index measured separately for normal subjects was 0.55.

### <<Example 3: Sensitivity and specificity of diagnosis for hemorrhagic stroke (subarachnoid hemorrhage and cerebral hemorrhage) by sCLEC2>>

When the threshold is set at 120 pg/mL, the diagnostic performance of sCLEC2 for hemorrhagic stroke is: sensitivity (34/34) = 100%, specificity (71/71) = 100%, and accuracy (105/105) = 100% (Table 5).

In conclusion, the measurement of sCLEC2, and the sCLEC2/platelet ratio (C2PAC index) can provide convenient and accurate data for the diagnosis of hemorrhagic stroke.

**[Table 5]**

| | Hemorrhagic stroke patients | Normal subjects | Total |
|---|---|---|---|
| sCLEC2 > 120 pg/mL | 34 | 0 | 34 |
| sCLEC2 < 120 pg/mL | 0 | 71 | 71 |
| Total | 34 | 71 | 105 |

### <<Example 4: Monitoring of patients with subarachnoid hemorrhage using sCLEC2>>

Various laboratory values on the first, eighth, and tenth hospital days of a case diagnosed with subarachnoid hemorrhage are shown (Figure 4). sCLEC2 increased slightly from the first day to the eighth day, but the C2PAC index showed a slight decreasing trend since the platelet counts also increased significantly.

### <<Example 5: Monitoring of patients with cerebral hemorrhage using sCLEC2>>

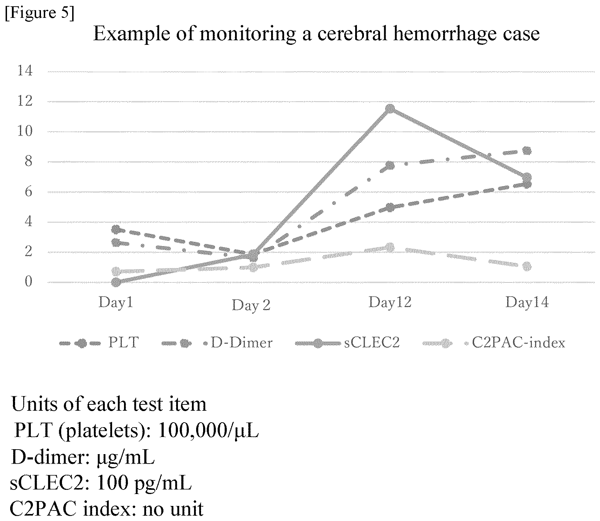

Various laboratory values on the first, second, twelfth, and fourteenth hospital days of a case diagnosed with cerebral hemorrhage are shown (Figure 5). sCLEC2 and the C2PAC index increased on the twelfth day, and decreased on the fourteenth day, while D-dimer increased from the twelfth day to the fourteenth day.

### INDUSTRIAL APPLICABILITY

As described above, the measured value of blood sCLEC2 and the ratio of sCLEC2/platelet in the present invention can be clinical tests for the diagnosis of hemorrhagic stroke, such as subarachnoid hemorrhage or cerebral hemorrhage, and can also be used to monitor the condition of patients with hemorrhagic stroke, such as subarachnoid hemorrhage or cerebral hemorrhage, and therefore, the measurement of sCLEC2 is useful as a method for evaluating the risk of hemorrhagic stroke patients.

## Claims

1. A method for evaluating a risk of hemorrhagic stroke, said method comprising:
measuring a concentration of soluble CLEC2 in blood collected from a patient suspected of having hemorrhagic stroke or a patient diagnosed with hemorrhagic stroke.

2. The method according to claim 1 for evaluating the risk of hemorrhagic stroke in a patient suspected of having hemorrhagic stroke or a patient diagnosed with hemorrhagic stroke, said method comprising:
(1) providing a blood sample derived from the patient;
(2) determining the concentration of soluble CLEC2 in the sample; and
(3) correlating the soluble CLEC2 concentration with the presence or absence of hemorrhagic stroke in the patient, or likelihood of outcome.

3. The method according to claim 2 for evaluating the risk of hemorrhagic stroke in a patient suspected of having hemorrhagic stroke or a patient diagnosed with hemorrhagic stroke, said step of correlating the soluble CLEC2 concentration with a presence or absence of hemorrhagic stroke in the patient, or likelihood of outcome comprising:
evaluating whether the patient is at risk based on a change in the soluble CLEC2 concentration.

4. The method according to claim 2 or 3 for evaluating the risk of hemorrhagic stroke in a patient suspected of having hemorrhagic stroke or a patient diagnosed with hemorrhagic stroke,
wherein, in the step of correlating the soluble CLEC2 concentration with hemorrhagic stroke, its threshold is 137 pg/mL.

5. The method according to claim 1 for evaluating the risk of hemorrhagic stroke in a patient suspected of having hemorrhagic stroke or a patient diagnosed with hemorrhagic stroke, said method comprising:
(1) providing a blood sample derived from the patient;
(2) determining the concentration of soluble CLEC2 in the sample;
(3) determining platelet counts in the sample;
(4) dividing the soluble CLEC2 concentration by the platelet counts; and
(5) correlating the value obtained by dividing the soluble CLEC2 concentration by the platelet counts, with a presence or absence of hemorrhagic stroke in the patient.

6. The method according to claim 5 for evaluating the risk of hemorrhagic stroke in a patient suspected of having hemorrhagic stroke or a patient diagnosed with hemorrhagic stroke, said method comprising:
wherein, in the step of correlating the value obtained by dividing the soluble CLEC2 concentration by the platelet counts with hemorrhagic stroke, a threshold of the value obtained by dividing the soluble CLEC2 concentration by the platelet counts is 0.7 to 1.0.

7. The method according to any one of claims 1 to 6, wherein the hemorrhagic stroke is either subarachnoid hemorrhage or cerebral hemorrhage.

8. The method according to any one of claims 1 to 7, wherein the step of determining the concentration of the soluble CLEC2 is performed by highly sensitive immunoassay, such as chemiluminescence immunoassay, electrochemiluminescence immunoassay, or fluorescence immunoassay.
